# EUROPÄISCHE PATENTSCHRIFT

(11) **EP 1 108 891 B1**
(45) Veröffentlichungstag und Bekanntmachung des Hinweises auf die Patenterteilung: **21.01.2009**
(21) Anmeldenummer: 00250424.9
(22) Anmeldetag: 08.12.2000
(51) Int. Cl.: F04B 43/12

(54) **Schlauchkassette für eine peristaltische Pumpe**
Tube cassette for a peristaltic pump
Cassette à tube de pompe péristaltique

(30) Priorität: 15.12.1999 DE 19960668
(43) Veröffentlichungstag der Anmeldung: 20.06.2001
(73) Patentinhaber: W.O.M. World of Medicine AG, 96337 Ludwigsstadt (DE)
(72) Erfinder: Haser, Christian, 14532 Stahnsdorf (DE); Zentner, Peter, 10961 Berlin (DE); Mehner, Gotthilf, 66280 Sulzbach (DE)
(74) Vertreter: Seuss, Thomas

(56) Entgegenhaltungen:
- US-A- 5 131 823
- US-A- 5 356 267
- US-A- 5 591 344

## Beschreibung

Die Erfindung betrifft eine Schlauchkassette für eine peristaltische Pumpe, mit einem Kassettengehäuse mit einem durch das Kassettengehäuse verlaufenden flexiblen Schlauch, wobei der Schlauch in dem Kassettengehäuse entlang eines Kreissegments geführt ist und wobei das Kassettengehäuse eine Ausnehmung zum Eingriff eines Rollenrades der peristaltischen Pumpe in das Innere des Kreissegments aufweist, sowie mit Verbindungselementen zum Anschluß des Kassettengehäuses an die peristaltische Pumpe. Die Erfindung betrifft weiterhin eine peristaltische Pumpe mit einer solchen Kassette. - Schlauchkassetten des eingangs genannten Aufbaus und peristaltische Pumpen werden insbesondere als Saug- und Spülpumpen für medizinische Anwendungen eingesetzt. Insofern kann der Schlauch die Funktion einer Spülleitung oder einer Saugleitung ausüben. Beispiele für medizinische Anwendungen sind die Arthroskopie, die Hysteroskopie und die Cystoskopie. Bei medizinischen Anwendungen ist insbesondere eine allen Anforderungen genügende Sterilität geboten und aus diesem Grunde wird der Schlauch üblicherweise nach einer Anwendung verworfen, um eine Kreuzkontaminationen zwischen verschiedenen Patienten zu vermeiden. Die Schlauchkassetten müssen folglich einerseits preiswert in der Herstellung sein und andererseits insbesondere eine einfache Handhabung ermöglichen.

Im Rahmen von bekannten peristaltischen Pumpen gibt es grundsätzlich zwei verschiedene Grundkonzeptionen. Die erste Grundkonzeption besteht darin, daß der um das Rollenrad angeordnete Schlauch mittels eines Andruckbügels o.dgl. gegen das Rollenrad gedrückt wird. Solche Ausführungsformen sind beispielsweise aus den Literaturstellen US-4,798,580 und US-5,044,902 bekannt. Von prinzipiell ähnlicher Funktionsweise ist der Gegenstand der Literaturstelle US-4,798,580, wonach eine Kassette mit einem Anpreßbügel hinter dem Schlauch vorgesehen ist. Mit einer Verschluß-Hebelmechanik wird die Kassette fixiert und gleichzeitig der Schlauch mittels des Anpreßbügels gegen das Rollenrad an dem Pumpengehäuse gedrückt. Beim Gegenstand der Literaturstelle US-5,044,902 ist das Rollenrad in die Kassette integriert und beim Einsetzen in die Pumpe wird der Schlauch durch die Zentrierung des Rollenrads auf seine Führung zur Kassettenwand hin und an diese angedrückt.

Die zweite Grundkonzeption von welcher die Erfindung im Kern ausgeht, besteht darin, daß der Schlauch durch eine Zugkraft geeigneter Größe mit genügendem Umfassungswinkel um das Rollenrad gezogen wird. Hierdurch wird ein Anpreßbügel o.dgl. entbehrlich. Die Zugkraft ist dabei in Abstimmung mit den elastischen Eigenschaften des Schlauches so zu dimensionieren, daß im Bereich einer Rolle eines Rollenrades der Innenquerschnitt des Schlauches auf praktisch Null reduziert ist. Beispiele hierfür sind in den Literaturstellen US-4,537,561 und US-5,213,483 beschrieben. Bei den insofern bekannten Ausführungsformen ist der Schlauch an einander gegenüberliegenden Enden der Kassette festgelegt und erstreckt sich zwischen den Enden geradlinig. Die Kassette wird dann in radialer Richtung, bezogen auf das Rollenrad, zum Rollenrad hin aufgedrückt und mit geeigneten Befestigungselementen befestigt. Hierdurch wird zwar durch Dimensionierung der Geometrie der Kassette sowie der Verbindungselementen nach Maßgabe der Elastizität des Schlauches eine den Anforderungen genügende Zugkraft auf den mit dem Rollenrad wechselwirkenden Schlauchteil eingerichtet, die Handhabung ist jedoch relativ umständlich und es ist regelmäßig erforderlich, daß beim Einsetzen der Schlauchkassette beide Hände verwendet werden. Dies liegt auch daran, daß die zum Aufdrücken der Kassette erforderlichen Kräfte durchaus beachtlich sein können. und bei manuellem Aufdrücken gegen diese Kräfte die exakte Positionierung einiges Geschick erfordert.

Demgegenüber liegt der Erfindung das technische Problem zugrunde, eine Schlauchkassette sowie eine peristaltische Pumpe anzugeben, welche den Anschluß der Kassette an ein Pumpengehäuse auf besonders einfach handhabbare Weise ermöglicht.

Zur Lösung dieses Problems lehrt die Erfindung, daß zumindest ein an das Kreissegment anschließender Schlauchschenkel in dem Kassettengehäuse in Längsrichtung des Schlauches zwischen einer Montageposition und einer Betriebsposition verschieblich gelagert und in der Betriebsposition fixierbar ist. Die Verschieblichkeit kann linear oder entlang eines Bogens eingerichtet sein. Die Grundkonzeption der Erfindung besteht darin, daß in einer ersten Montagehandlung die Kassette mit in Montageposition befindlichem Schlauchschenkel an ein Pumpengehäuse angesetzt wird und im zweiten Montageschritt durch Zug an dem verschieblichen Schlauchschenkel der Schlauch sich an das Rollenrad mit dem erforderlichen Druck anlegt. Dies ist dann die Betriebsposition des Schlauchschenkels. Hierdurch wird erreicht, daß die Schlauchkassette auf einfache Weise an einem Pumpengehäuse angebracht werden kann, und zwar ohne Gegendruck aus elastischen Kräften des Schlauches. Die Kassette läßt sich also unschwer einhändig anbringen. Hieran unmittelbar anschließend wird die erforderliche Anlage des Schlauches um das Rollenrad mit ausreichender Zugkraft durch einfachen Zug an dem verschieblichen Schlauchschenkel eingerichtet. Es versteht sich, daß der verschiebliche Schlauchschenkel in der Betriebsposition auf geeignete Weise fixierbar ist, beispielsweise rastend. Auch hierfür wird lediglich eine Hand benötigt, da das Gewicht der peristaltischen Pumpe eine ausreichende Gegenkraft gegen die elastischen Kräfte des Schlauches aufbaut.

Bevorzugt ist es, wenn die Betriebsposition dadurch einstellbar ist, daß bei an der peristaltischen Pumpe angebrachter Kassette der verschiebliche Schlauchschenkel durch Zug an dem unmittelbar an den verschieblichen Schlauchschenkel anschließenden und außerhalb der Kassette angeordneten Schlauchteil einstellbar ist, wobei der verschiebliche Schlauchschenkel in der Betriebsposition einrastet. Grundsätzlich können beide Schlauchschenkel verschieblich gelagert sein. Im Zusammenhang mit weiteren gegebenenfalls einzurichtenden Funktionen empfiehlt es sich jedoch, daß nur ein Schlauchschenkel zwischen der Montageposition und der Betriebsposition verschieblich ist und daß der andere Schlauchschenkel in Längsrichtung des Schlauches fixiert ist.

Grundsätzlich ist der Umfassungswinkel des Schlauches um das Rollenrad beliebig, solange eine hinreichende Querschnittsreduktion bei entsprechender Zugkraft auf den Schlauch im Bereich einer Rolle entsteht. Bevorzugt ist es, daß das Kreissegment und somit in etwa der Umfassungswinkel des Schlauches um das Rollenrad sich über zumindest 90°, vorzugsweise 170° bis 190°, höchst vorzugsweise 180°, erstreckt. Zweckmäßig ist es, den Schlauch zumindest im Bereich des Kreissegments aus einem gummielastischen Werkstoff, insbesondere einem natürlichen oder synthetischen Elastomer, vorzugsweise einem Silikonkautschuk oder einem elastischen Polyurethan(co)polymeren, gebildet ist. Je höher das Elastizitätsmodul ist, um so niedriger ist die erforderliche Zugkraft an dem verschieblichen Schlauchschenkel.

In der Ausführungsform mit lediglich einem verschieblichen Schlauchschenkel ist es bevorzugt, daß der fixierte Schlauchschenkel ein Verbindungselement mit vorzugsweise orthogonal zur Längserstreckung des Schlauches verlaufenden Anschlagflächen aufweist, wobei die Anschlagflächen gegen Fixierflächen in dem Kassettengehäuse anlaufen. Durch Wechselwirkung der Anschlagflächen mit den Fixierflächen ist das Verbindungsstück und somit der Schlauchschenkel praktisch ohne Spiel fixiert. Diese Ausführungsform ermöglicht es, daß das Verbindungselement des fixierten Schlauchschenkels einen Drucksensor aufweist, welcher bei an der peristaltischen Pumpe angebrachter Kassette mit der peristaltischen Pumpe kommuniziert. Hierbei kann der Drucksensor, welcher in hydrostatischer Verbindung mit einem durch den Schlauch fließenden Fluid steht, als Druckwandler ausgebildet sein, wobei der auf den Sensor wirkende Druck in eine elektrische Größe bzw. ein elektrisches Signal umgewandelt wird. Die Kommunikation mit der peristaltischen Pumpe funktioniert dann über zueinander komplementäre Kontakte an dem Drucksensor sowie an der peristaltischen Pumpe. Es ist aber auch möglich, daß der Drucksensor als Druckmembran ausgebildet ist und mit einem im Rahmen der peristaltischen Pumpe eingerichteten Druckwandler bei montierter Schlauchkassette in mechanischer Verbindung steht. Dies ist insbesondere in Kostenhinsicht vorteilhaft.

Vorzugsweise ist der verschiebliche Schlauchschenkel mit einem Verbindungselement ausgestattet, welches ein Rastelement aufweist. Im einzelnen kann das Rastelement eine Anlauffläche aufweisen, mittels welcher bei an der peristaltischen Pumpe angebauter Kassette ein an der peristaltischen Pumpe angeordnetes Sperrelement im Zuge der Verschiebung des verschieblichen Schlauchschenkels aus der Montageposition in die Betriebsposition gegen Federdruck aus einer Sperrstellung aushebbar ist, wobei das Rastelement in Richtung der Betriebsposition an die Anlauffläche anschließend eine Sperrfläche aufweist, hinter welche das Sperrelement in seiner Sperrstellung greift. Der besondere Vorteil dieser speziellen Ausführungsform liegt darin, daß aufgrund der Anordnung der Sperrfläche einerseits im Rahmen der Kassette sowie andererseits des Sperrelements im Rahmen der peristaltischen Pumpe beim Entfernen der Kassette von dem Pumpengehäuse kein besonderer Entriegelungsmechanismus oder keine besondere Entriegelungshandhabung erforderlich ist. Beim Abziehen der Kassette von dem Pumpengehäuse kommt das Sperrelement vielmehr von der Sperrfläche frei mit der Folge der Freigabe des Verbindungselementes und somit der Entspannung des Schlauches durch Verschiebung des verschieblichen Schlauchschenkels in die Montageposition. Im einfachsten Fall ist das Sperrelement als Sperrstift ausgeführt.

Bevorzugt ist es, wenn der Schlauch, gegebenenfalls einschließlich der Verbindungselemente, dem Kassettengehäuse entnehmbar ist. Im einzelnen kann in diesen Zusammenhängen das Kassettengehäuse von im wesentlichen halbovaler Form mit zwei ebenen Halbovalflächen sein, wobei eine Halbovalfläche geschlossen und wobei die gegenüberliegende Halbovalfläche offen ist und eine Ausnehmung zum Eingriff des Rollenrades aufweist. Durch die offene Halbovalfläche kann der Schlauch, gegebenenfalls mit seinen Verbindungselementen, entnommen werden. Eine solche Ausführungsform erlaubt eine Wiederverwendung des Kassettengehäuses und somit eine beachtliche Reduktion des Abfallanfalls. Hierbei wird dann eine verworfene Kassette als Abfall gesammelt und beispielsweise dem Hersteller wieder zugeführt, welcher dann auf einfache Weise den Schlauch sowie die Verbindungselemente der Kassette entnimmt, verwirft und das Kassettengehäuse einer erneuten Nutzung zuführt.

Die Verbindung der Schlauchkassette mit der peristaltischen Pumpe kann auf die verschiedensten Weisen erfolgen. In jedem Fall handelt es sich um eine lösbare Verbindung. Eine bevorzugte Ausführungsform ist dadurch gekennzeichnet, daß an einer orthogonal zu den Halbovalflächen stehenden Wandung zumindest eine Formschlußausnehmung eingerichtet ist, und daß in Abstand, bezogen auf die Wandung, zu der Formschlußausnehmung zumindest ein Kraftschlußverbindungselement eingerichtet ist, wobei mittels des Kraftschlußverbindungselements eine lösbare Verbindung mit der peristaltischen Pumpe durch Bewegung des Kraftschlußverbindungselementes in Richtung parallel zur Wandung herstellbar ist. In dieser Ausführungsform erfolgt der Einsatz wie folgt. Eine Bedienperson greift das Kassettengehäuse und setzt dieses schräg an der peristaltischen Pumpe unter Herstellung einer Formschlußverbindung an. Nach Herstellung der Formschlußverbindung wird die Kassette zur peristaltischen Pumpe hin kippend (Drehachse bei der Formschlußverbindung) angedrückt, wobei die Kraftschlußverbindung hergestellt und die Kassette so sicher positioniert an dem Pumpengehäuse gehalten wird.

Die Erfindung betrifft weiterhin eine peristaltische Pumpe, mit einem Pumpengehäuse, mit einem über eine Außenwandung des Pumpengehäuses hervorragenden Rollenrad sowie mit einer erfindungsgemäßen Kassette, wobei an der Außenwandung zu den Verbindungselementen der Kassette komplementäre Anschlußelemente eingerichtet sind. Es versteht sich hierbei, daß die Drehachse des Rollenrades orthogonal zur Außenwandung sowie im wesentlichen koaxial zum Kreissegment steht. Im Rahmen einer erfindungsgemäßen peristaltischen Pumpe können im Zusammenhang mit der Schlauchkassette diverse weitere Funktionen eingerichtet sein. Die Anordnung eines Druckwandlers, welcher mit einer Druckmembran bei angesetzter Kassette in Wechselwirkung steht, ist bereits vorstehend beschrieben worden. Weiterhin ist es möglich, daß ein Antriebsmotor des Rollenrades so gesteuert wird, daß ein Antrieb nur bei angesetzter Kassette möglich ist. Dies läßt sich beispielsweise durch einen im Zuge des Ansetzens der Kassette an das Pumpengehäuse betätigbaren Schalter o.dgl. bewirken. Eine besonders vorteilhafte Ausführungsform ist dadurch gekennzeichnet, daß ein Schaltelement zur Steuerung des Antriebsmotors des Rollenrades durch das Sperrelement betätigbar ist. Hierbei ist das Schaltelement in der Sperrstellung des Sperrelements aktiviert, nicht jedoch in einer sich ohne angesetzte Kassette einstellenden Ruhestellung. Im Ergebnis kann das Rollenrad nur dann angesteuert bzw. rotierend angetrieben werden, wenn (kumulativ) die Kassette an das Pumpengehäuse angesetzt ist und der verschiebliche Schlauchschenkel sich in der Betriebsposition befindet.

Im folgenden wird die Erfindung anhand von lediglich eine Ausführungsbeispiel darstellenden Figuren näher erläutert. Es zeigen:
- Fig. 1: eine erfindungsgemäße Schlauchkassette in Gesamtansicht,
- Fig. 2: eine erfindungsgemäße peristaltische Pumpe,
- Fig. 3a, b: eine Detailansicht im Bereich eines verschieblichen Schlauchschenkels bei an der peristaltische Pumpe angesetzter Kassette, und zwar in Montageposition (a) sowie Betriebsposition (b), des Schlauchschenkels,
- Fig. 4a,b: ein Verbindungselement für einen verschieblichen Schlauchschenkel in zwei verschiedenen Ansichten sowie
- Fig. 5: eine alternative Ausführungsform zum Gegenstand der Fig. 4.

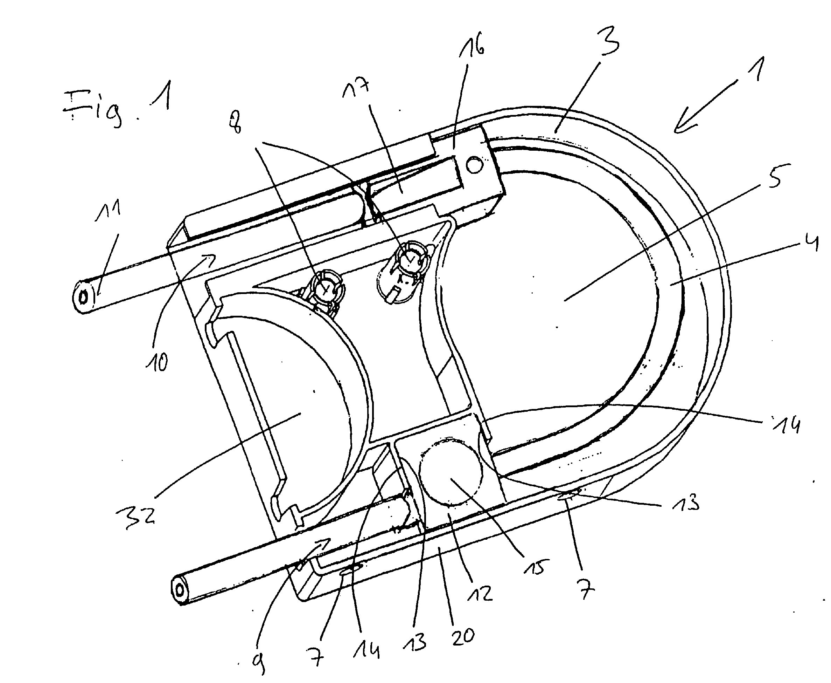

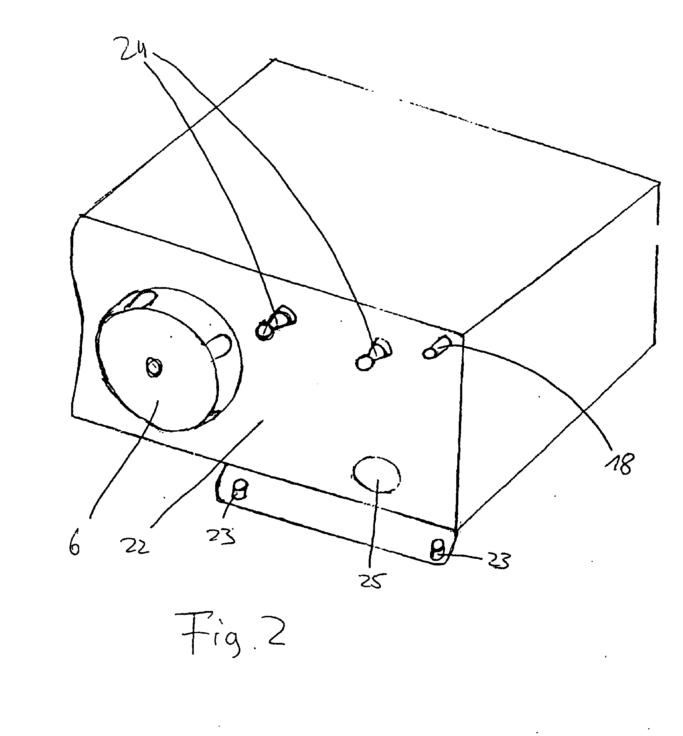

In der Fig. 1 erkennt man eine Schlauchkassette 1 für eine peristaltische Pumpe 2 (siehe auch Fig. 2) mit einem Kassettengehäuse 3, mit einem durch das Kassettengehäuse 3 verlaufenden flexiblen Schlauch 4, wobei der Schlauch 4 in dem Kassettengehäuse 3 entlang eines Kreissegments, im Ausführungsbeispiel ca. 180°, geführt ist. Das Kassettengehäuse 3 ist von in wesentlicher halbovaler Form mit zwei ebenen Halbovalflächen, wobei eine Halbovalfläche geschlossen und wobei die gegenüberliegende Halbovalfläche offen ist und somit eine Ausnehmung 5 zum Eingriff des Rollenrades 6 aufweist. Das Rollenrad 6 der peristaltischen Pumpe 2 greift bei montierter Schlauchkassette 1 in das Innere des Kreissegmentes ein. In der Fig. 1 erkennt man weiterhin Verbindungselemente 7, 8 zum Anschluß des Kassettengehäuses 3 an die peristaltische Pumpe 2. In der Fig. 2 ist zu erkennen, daß die das Rollenrad tragende Außenwandung 22 des Pumpengehäuses 21 zu den Verbindungselementen 7, 8 der Kassette komplementärer Anschlußelemente 23, 24 aufweist. In der Fig. 2 erkennt man weiterhin, daß das Rollenrad 6 über die Außenwandung 22 des Pumpengehäuses hervorragt. Bei montierter Schlauchkassette 1 ist die Drehachse des Rollenrades 6 im wesentlichen koaxial zum Kreissegment der Führung des Schlauches 4 in dem Kassettengehäuse 3, wie eine vergleichende Betrachtung der Fig. 1 und 2 verdeutlichet.

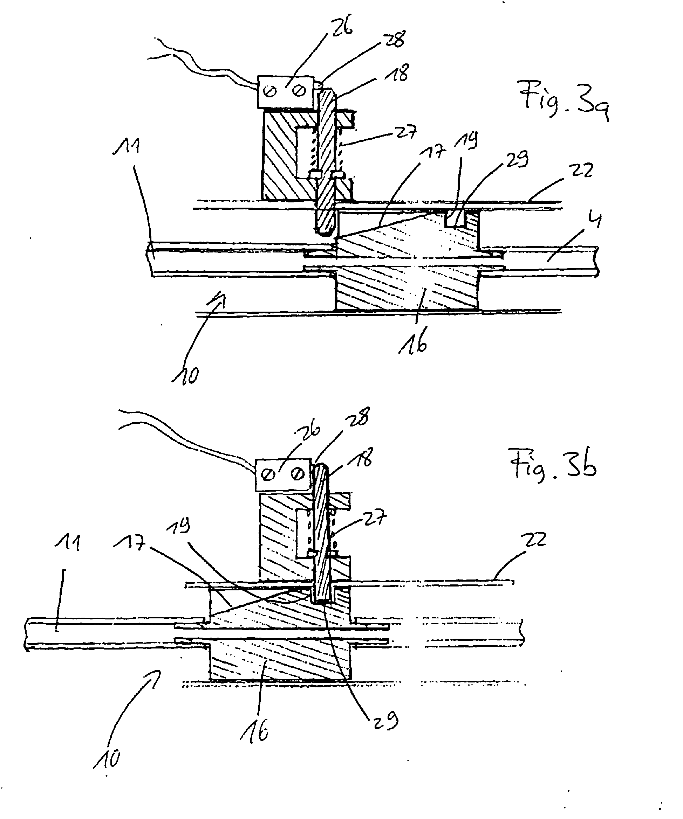

Der Fig. 1 entnimmt man, daß ein an das Kreissegment anschließender Schlauchschenkel 10 zwischen einer Montageposition und einer Betriebsposition verschieblich gelagert ist. Die Montageposition und die Betriebsposition sind hierbei im Detail insbesondere einer Betrachtung der Fig. 3a und 3b zu entnehmen. Der Fig. 3 entnimmt insbesondere, daß ein Verbindungselement 16 mit einem Rastelement im Rahmen des verschieblichen Schlauchschenkels 10 eingerichtet ist. Das Rastelement ist mit einer Anlauffläche 17 ausgestattet, mittels welcher bei an der peristaltischen Pumpe 2 angebrachter Kassette 1 ein an der peristaltischen Pumpe 2 angeordnetes Sperrelement 18, ein Sperrstift 18, im Zuge der Verschiebung des verschieblichen Schlauchschenkels 10 aus der Montageposition in die Betriebsposition gegen Federdruck aus einer Ruhestellung aushebt. Das Rastelement weist in Richtung der Betriebsposition an die Anlauffläche 17 anschließend eine Sperrfläche 19 auf, hinter welche das Sperrelement 18 in seiner Sperrstellung greift. In der vergleichenden Betrachtung der Fig. 3a und 3b entnimmt man weiterhin, daß im Rahmen der peristaltischen Pumpe 2 ein Schaltelement 26 eingerichtet ist, welches mit dem Sperrstift 18 wechselwirkt. In der Fig. 3a ist der Sperrstift 18 in seiner Ruhestellung über seine Sperrstellung hinaus aus der Außenwandung 22 des Pumpengehäuses 21 hervorgetreten unter Krafteinwirkung der Druckfeder 27. Hierbei ist durch geeignete Anordnung des Schaltelements 26 ein Betätigungselement 28 des Schaltelements 26 freigegeben. Im Zuge der Verschiebung des Verbindungselementes 16 von der Darstellung in Fig. 3a in die Darstellung der Fig. 3b wird der Sperrstift 18 gegen die Federkraft der Druckfeder 27 in Richtung innerhalb der Außenwandung 22 des Pumpengehäuses 21 verschoben durch Einwirkung der Anlauffläche 17. Im Zuge dieser Bewegung wird das Betätigungselement 28 des Schaltelements 26 betätigt. Sobald die Betriebsposition (Fig. 3b) erreicht ist, fällt der Sperrstift 18 in eine Ausnehmung mit einer Sperrfläche 19 und somit in seine Sperrstellung. Hierdurch wird der Schlauch 4 entgegen seiner inneren elastischen Kräfte in der Betriebsposition gehalten. Von Bedeutung ist in diesen Zusammenhängen, daß der Sperrstift 18 zusätzlich auf einem Grund 29 der Ausnehmung aufschlägt, wobei Anordnung des Schaltelementes 26, Länge des Sperrstifts 18 sowie Anordnung des Grundes 29 mit der Maßgabe getroffen sind, daß das Betätigungselement 28 in der Sperrstellung des Sperrstifts 18 betätigt bleibt. Im Ergebnis ist über das Schaltelement 26 das Rollenrad 6 nur in Betriebsposition des Schlauchschenkels 10 möglich.

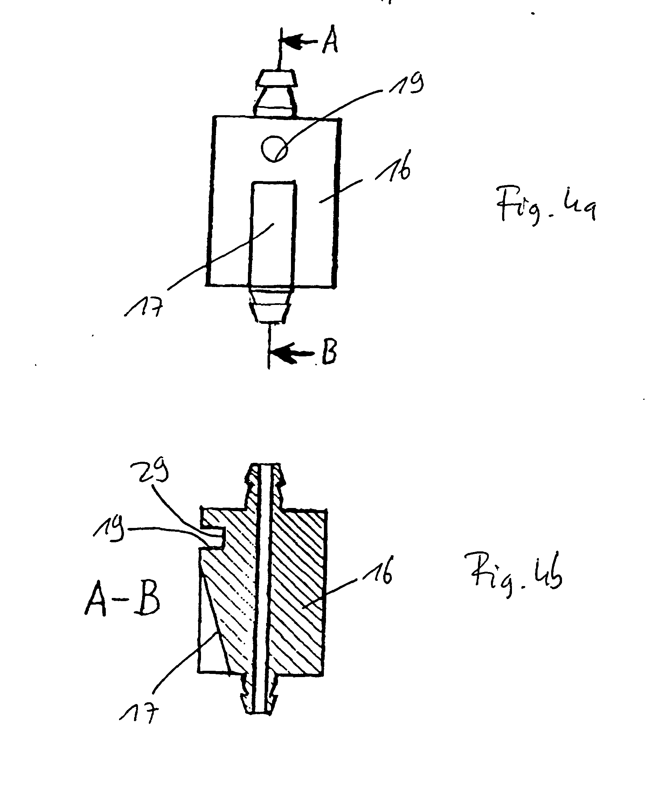

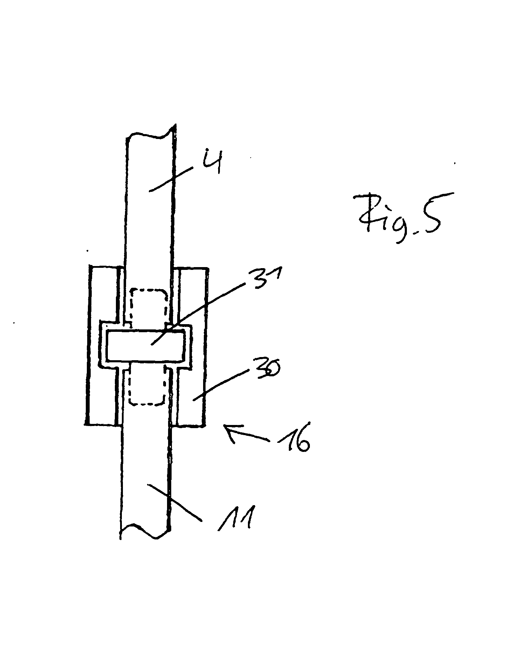

In den Fig. 4a und 4b ist das Verbindungselement 16 des verschieblichen Schlauchschenkels 10 im Detail aus zwei zueinander orthogonalen Ansichten dargestellt. Man erkennt hier wiederum die Anlauffläche 17, die Sperrfläche 19 sowie den Grund 29. In diesem Ausführungsbeispiel ist das Verbindungselement 16 einstückig ausgeführt. Es ist aber auch eine zwei bzw. mehrstückige Ausführungen möglich, wie bspw. in der Fig. 5 gezeigt. Beim Gegenstand der Fig. 5 ist ein Verbinder 31 in Richtung der Längserstreckung des Schlauches 4 fixierbar in ein Verbindungsstückgehäuse 30 eingelegt. Gegenüberliegend der Aufsicht der Fig. 5 weist das Verbindungsstückgehäuse 30 einen Aufbau entsprechend der Darstellung in Fig. 4a auf.

Der Fig. 1 entnimmt man, daß der zweite Schlauchschenkel 9 in Längsrichtung des Schlauches 4 fixiert ist. Im Rahmen des fixierten Schlauchschenkels 9 ist ein Verbindungselement 12 mit orthogonal zur Längserstreckung des Schlauches 4 verlaufenen Anschlagflächen 13 ausgebildet. Die Anschlagflächen 13 laufen gegen Fixierflächen 14 in dem Kassettengehäuse 2 an. Im Rahmen des Verbindungselements 12 des fixierten Schlauchschenkels 9 ist ein Drucksensor 15 eingerichtet, welcher bei an der peristaltischen Pumpe 2 angebrachter Kassette 1 mit der peristaltischen Pumpe 2 kommuniziert. Im Ausführungsbeispiel ist der Drucksensor 15 als Druckmembran 15 einer Druckkammer ausgebildet und am Pumpengehäuse 21 ist ein Druckwandler 25 mit der Maßgabe angebracht, daß die Druckmembran 15 bei montierter Kassette 1 am Druckwandler 25 anliegt (siehe auch Fig. 2). Der Fig. 1 entnimmt man, daß der Schlauch 4 einschließlich der Verbindungselemente 12, 16 dem Kassettengehäuse 3 entnehmbar ist. Die Entnahme erfolgt hierbei in Richtung etwa orthogonal nach oberhalb der Papierebene in der dargestellten Ansicht.

Der Schlauch 4 besteht im Bereich des Kreissegments bzw. zwischen den Verbindungselementen 12, 16 aus einem gummielestischen Werkstoff, nämlich Silikonkautschuk. Der Schlauch 4 kann gegenüberliegend anschließend an den Verbindungselementen 12, 16 aus dem gleichen oder einem hiervon verschiedenen Werkstoff bestehen. Insbesondere ist es möglich, daß insofern auch nicht gummielastische Werkstoffe verwendet werden.

Einer vergleichenden Betrachtung der Fig. 1 und 2 entnimmt man die Mittel zur Befestigung der Schlauchkassette 1 an der peristaltischen Pumpe 2. In der Fig. 1 ist ersichtlich, daß an einer orthogonal zu den Halbovalflächen stehenden Wandung 20 zwei Formschlußausnehmungen 7 eingerichtet sind, und daß in Abstand bezogen auf die Wandung 20 zu den Formschlußausnehmungen 7 zwei Kraftschlußverbindungselemente 8 eingerichtet sind. Im Rahmen der peristaltischen Pumpe sind komplementäre Anschlußelemente 23, 24 eingerichtet. Bei den Anschlußelementen 23 handelt es sich um in die Formschlußausnehmungen 7 formschlüssig eingreifende Haltestifte 23. Die Kraftschlußverbindungselemente 8 sowie die hierzu komplementären Anschlußnippel 24 sind als lösbare Kraftschlußverbindung ausgebildet. Zum Ansetzen der Schlauchkassette 1 an die peristaltische Pumpe 2 werden zunächst die Formschlußausnehmungen 7 auf die Haltestifte 23 positioniert und aufgeschoben, wobei die Schlauchkassette 1 gegenüber der Außenwandung 22 abgekippt gehalten wird. Es versteht sich, daß zwischen den Formschlußausnehmungen 7 und den Haltestiften 23 ein hierfür ausreichendes Spiel vorgesehen ist. Nach dem Aufsetzen wird die Schlauchkassette 1 gegen die Außenwandung 22 gekippt und angedrückt, wodurch die Verbindung zwischen den Kraftschlußverbindungselementen 8 und Anschlußnippeln 24 hergestellt und die Schlauchkassette 1 an der peristaltischen Pumpe 2 gehalten wird. Die Abnahme der Schlauchkassette erfolgt in umgekehrter Richtung.

Im Ausführungsbeispiel funktioniert der Schlauch 4 als Spülleitung. Ebenso kann der Schlauch 4 aber auch als Saugleitung funktionieren. Weiterhin ist es, unabhängig vom Ausfuhrunsbeispiel, möglich, daß im Rahmen der Kassette ein zweiter Schlauch eingerichtet ist, beispielsweise als Ablaßleitung funktionierend, wobei im Rhamen der peristalitischen Pumpe Absperrmittel eingerichtet sein können, welche bei angesetzter Kassette den zweiten Schlauch absperren. Im Ausführungsbeispiel gemäß Fig. 1 ist hierzu eine Kassettenkammer 32 vorgesehen.

## Patentansprüche

1. Schlauchkassette (1) für eine peristaltische Pumpe (2) mit einem Kassettengehäuse (3), mit einem durch das Kassettengehäuse (3) verlaufenden flexiblen Schlauch (4), wobei der Schlauch (4) in dem Kassettengehäuse (3) entlang eines Kreissegments geführt ist, wobei das Kassettengehäuse (3) eine Ausnehmung (5) zum Eingriff eines Rollenrades (6) der peristaltischen Pumpe (2) in das Innere des Kreissegments aufweist, sowie mit Verbindungselementen (7), (8) zum Anschluß des Kassettengehäuses (3) an die peristaltische Pumpe (2),
**dadurch gekennzeichnet,**
**daß** zumindest ein an das Kreissegment anschließender Schlauchschenkel (9), (10) in dem Kassettengehäuse (3) in Längsrichtung des Schlauches (4) zwischen einer Montageposition und einer Betriebsposition verschieblich gelagert
und in der Betriebsposition fixierbar ist.

2. Schlauchkassette (1) nach Anspruch 1, **dadurch gekennzeichnet, daß** der verschiebliche Schlauchschenkel durch ein Rastelement fixierbar ist.

3. Schlauchkassette (1) nach Anspruch 1, **dadurch gekennzeichnet, daß** die Betriebsposition dadurch einstellbar ist, daß bei an einer peristaltischen Pumpe (2) angebrachter Kassette (1) der verschiebliche Schlauchschenkel (10) durch Zug an dem unmittelbar an den verschieblichen Schlauchschenkel (10) anschließenden und außerhalb der Kassette angeordneten Schlauchteil (11) einstellbar ist, wobei der verschiebliche Schlauchschenkel (10) mittels eines Rastelementes in der Betriebsposition durch Einrasten fixiert wird.

4. Schlauchkassette (1) nach Anspruch 1, 2 oder 3, **dadurch gekennzeichnet, daß** nur ein Schlauchschenkel (10) zwischen der Montageposition und der Betriebsposition verschieblich ist, und daß der andere Schlauchschenkel (9) in Längsrichtung des Schlauchendes (4) fixiert ist.

5. Schlauchkassette (1) nach einem der Ansprüche 1 bis 4, **dadurch gekennzeichnet, daß** das Kreissegment sich über zumindest 90°, vorzugsweise 170° bis 190°, höchst vorzugsweise 180°, erstreckt.

6. Schlauchkassette (1) nach einem der Ansprüche 1 bis 5, **dadurch gekennzeichnet, daß** der Schlauch (4) zumindest im Bereich des Kreissegments aus einem gummielastischen Werkstoff, insbesondere einem natürlichen oder synthetischen Elastomer, vorzugsweise einem Silikonkautschuk oder einem elastomeren Polyurethan(co)polymeren, gebildet ist.

7. Schlauchkassette (1) nach einem der Ansprüche 1 bis 6, **dadurch gekennzeichnet, daß** der fixierte Schlauchschenkel (9) ein Verbindungselement (12) mit vorzugsweise orthogonal zur Längserstreckung des Schlauches (4) verlaufenden Anschlagsflächen (13) aufweist, wobei die Anschlagflächen (13) gegen Fixierflächen (14) in dem Kassettengehäuse (2) anlaufen.

8. Schlauchkassette (1) nach einem der Ansprüche 1 bis 7, **dadurch gekennzeichnet, daß** das Verbindungselement (12) des fixierten Schlauchschenkels (9) einen Drucksensor (15) aufweist, welcher bei an der peristaltischen Pumpe (2) angebrachter Kassette (1) mit der peristaltischen Pumpe (2) kommuniziert.

9. Schlauchkassette (1) nach einem der Ansprüche 2 bis 8,
**dadurch gekennzeichnet, daß** der verschiebliche Schlauchschenkel (10) ein Verbindungselement (16) mit einem Rastelement aufweist.

10. Schlauchkassette (1) nach Anspruch 9,
**dadurch gekennzeichnet, daß** das Rastelement eine Anlauffläche (17) aufweist, mittels welcher bei an der peristaltischen Pumpe (2) angebrachte Kassette (1) ein an der peristaltischen Pumpe (2) angeordnete Sperrelement (18) im Zuge der Verschiebung des verschieblichen Schlauchschenkels (10) aus der Montageposition in die Betriebsposition gegen Federdruck aus einer Sperrstellung aushebbar ist, und daß das Rastelement in Richtung der Betriebsposition an die Anlauffläche (17) anschließend eine Sperrfläche (19) aufweist, hinter welche das Sperrelement (18) in seiner Sperrstellung greift.

11. Schlauchkassette (1) nach Anspruch 10,
**dadurch gekennzeichnet, daß** das Sperrelement (18) als Sperrstift (18) ausgeführt ist.

12. Schlauchkassette (1) nach einem der Ansprüche 1 bis 11, **dadurch gekennzeichnet, daß** der Schlauch (4), gegebenenfalls einschließlich der Verbindungselemente (12, 16), dem Kassettengehäuse (3) entnehmbar ist.

13. Schlauchkassette nach einem der Ansprüche 1 bis 12,
**dadurch gekennzeichnet, daß** das Kassettengehäuse (3) von im wesentlichen halbovaler Form mit zwei Ebenen Halbovalflächen ist, wobei eine Halbovalfläche geschlossen und wobei die gegenüberliegende Halbovalfläche offen ist und die Ausnehmung (5) zum Eingriff des Rollenrades (6) aufweist.

14. Schlauchkassette (1) nach Anspruch 13,
**dadurch gekennzeichnet, daß** an einer orthogonal zu den Halbovalflächen stehenden Wandung (20) zumindest eine Formschlußausnehmung (7) eingerichtet ist und daß im Abstand, bezogen auf die Wandung (20) zu der Formschlußausnehmung (7) zumindest ein Kraftschlußverbindungselement (8) eingerichtet ist, wobei mittels des Kraftschlußverbindungselements (8) eine lösbare Verbindung mit der peristaltischen Pumpe (2) durch Bewegung des Kraftschlußverbindungselements (8) in Richtung parallel zur Wandung (20) herstellbar ist.

15. Peristaltische Pumpe (2) mit einem Pumpengehäuse (21), mit einem über eine Außenwandung (22) des Pumpengehäuses (21) hervorragenden Rollenrad (6) sowie mit einer Kassette (1) nach einem der Ansprüche 1 bis 13, wobei an der Außenwandung (22) zu den Verbindungselementen (7, 8) der Kassette komplementäre Anschlußelemente (23, 24) eingerichtet sind.

16. Peristaltische Pumpe (2) nach Anspruch 15, wobei der Drucksensor (15) als Druckkammer mit Druckmembran (15) ausgebildet ist und wobei am Pumpengehäuse (21) ein Druckwandler (25) mit der Maßgabe angebracht ist, daß die Druckmembran (15) bei montierter Kassette (1) am Druckwandler (25) anliegt.

## Claims

1. A hose cassette (1) for a peristaltic pump (2) including a cassette housing (3), a flexible hose (4) extending through the cassette housing (3), the hose (4) being guided in the cassette housing (3) along a circular segment, the cassette housing (3) having a cutout (5) for the engagement of a roller wheel (6) of the peristaltic pump (2) into the interior of the circular segment, and also including connection elements (7, 8) for connecting the cassette housing (3) to the peristaltic pump (2),
**characterized in**
**that** at least one hose leg (9), (10) to be connected to the circular segment is displaceably held between a mounting position and an operating position in the cassette housing (3) in the longitudinal direction of the hose (4)
and is fixable in the operating position.

2. The hose cassette (1) according to claim 1, **characterized in that** the displaceable hose leg is fixable by a latch element.

3. The hose cassette (1) according to claim 1, **characterized in that** the operating position is adjustable by that for the cassette (1) mounted at a peristaltic pump (2), the displaceable hose leg (10) is adjustable by pulling at the hose portion (11) immediately adjacent to the displaceable hose leg (10) and being arranged outside of the cassette, the displaceable hose leg (10) being fixed in said operating position by a latch element.

4. The hose cassette (1) according to claim 1, 2 or 3, **characterized in that** only one hose leg (10) is displaceable between the mounting position and the operating position, and that the other hose leg (9) is fixed in longitudinal direction of the end portion of the hose (4).

5. The hose cassette (1) according to one of claims 1 to 4, **characterized in that** the circular element extends over at least 90°, preferably over 170° to 190°, most preferably over 180°.

6. The hose cassette (1) according to one of claims 1 to 5, **characterized in that** the hose (4) is formed at least in an area of said circular segment from a rubber-elastic material, in particular from a natural or synthetic elastomer material, preferably a silicone rubber or an elastomer polyurethane-(co)polymer.

7. The hose cassette (1) according to one of claims 1 to 6, **characterized in that** the fixed hose leg (9) comprises a connection element (12) having stop surfaces (13) preferably extending orthogonally to a longitudinal extension of the hose (4), said stop surfaces (13) resting against fixing surfaces (14) in said cassette housing (2).

8. The hose cassette (1) according to one of claims 1 to 7, **characterized in that** the connection element (12) of said fixed hose leg (9) comprises a pressure sensor (15) communicating with the peristaltic pump (2), for the cassette (1) mounted at the peristaltic pump (2).

9. The hose cassette (1) according to one of claims 2 to 8, **characterized in that** the displaceable hose leg (10) comprises a connection element (16) with a latch element.

10. The hose cassette (1) according to claim 9, **characterized in that** said latch element has a stop surface (17) for lifting a locking element (18), for the cassette (1) mounted at the peristaltic pump (2), during the displacement of displaceable hose leg (10) from the mounting position into the operating position against a spring pressure from the locking position, and that said latch element comprises adjacent to the stop surface (17) a blocking surface (19) in the direction of the operating position, said locking element engaging in its locking position behind the locking element (18).

11. The hose cassette (1) according to claim 10, **characterized in that** the locking element (18) is configured as a locking pin (18).

12. The hose cassette (1) according to one of claims 1 to 11, **characterized in that** the hose (4), if applicable together with the connection elements (12, 16), is removably mounted in the cassette housing (3).

13. The hose cassette (1) according to one of claims 1 to 12, **characterized in that** the cassette housing (3) has a substantially half-oval shape with two plane half-oval surfaces, one half-oval surface being closed and the opposite half-oval surface being open and comprising the cutout (5) for engagement of the roller wheel (6).

14. The hose cassette (1) according to claim 13, **characterized in that** at least one form fit cutout (7) is provided at a wall (20) orthogonal to said half-oval surfaces, and that spaced from the wall (20) to the form fit cutout (7), at least one friction element (8) is provided, by means of the friction element (8) a releasable connection to the peristaltic pump (2) being established by moving the friction element (8) in a direction parallel to the wall (20).

15. A peristaltic pump (2) comprising a pump housing (21), a roller wheel (6) projecting from an outside wall (22) of the pump housing (21) and a cassette (1) according to one of claims 1 to 13, wherein connection elements (23, 24) are provided at the outside wall (22), said connection elements being complementary to the cassette connection elements (7, 8).

16. A peristaltic pump (2) according to claim 15, wherein the pressure sensor (15) is configured as a pressure chamber with a pressure membrane (15), and wherein a pressure transducer (25) is mounted at the pump housing (21) such that with mounted cassette (1), the pressure membrane (15) will rest against the pressure transducer (25).

## Revendications

1. Cassette de tube (1) pour une pompe péristaltique (2) comprenant un boîtier de la cassette (3), un tube flexible (4) s'étendant à travers le boîtier de la cassette (3), le tube (4) étant guidé dans le boîtier de la cassette (3) le long d'un segment circulaire, le boîtier de la cassette (3) ayant un évidement (5) pour l'accrochage d'une roue à galet (6) de la pompe péristaltique (2) dans l'intérieur du segment circulaire, et aussi comprenant des éléments de jonction (7, 8) pour joindre le boîtier de la cassette (3) à la pompe péristaltique (2),
**caractérisée en ce**
**qu'**au moins une branche du tube (9), (10) voisine au segment circulaire est maintenue de manière amovible entre une position de montage et une position de fonctionnement dans le boîtier de la cassette (3) dans la direction longitudinale du tube (4),
et est fixable dans la position de fonctionnement.

2. Cassette de tube (1) selon la revendication 1, **caractérisée en ce que** la branche amovible du tube est fixable par un élément d'accrochage.

3. Cassette de tube (1) selon la revendication 1, **caractérisée en ce que** la position de fonctionnement est réglable **en ce que** pour la cassette (1) montée à une pompe péristaltique (2), la branche amovible du tube (10) est réglable par tirage à la partie du tube (11) immédiatement voisine à la branche amovible du tube (10) et étant arrangée à l'extérieur de la cassette, la branche amovible du tube (10) étant fixée dans cette position de fonctionnement par un élément d'accrochage.

4. Cassette de tube (1) selon la revendication 1, 2 ou 3, **caractérisée en ce que** seulement une seule branche du tube (10) est amovible entre la position de montage et la position de fonctionnement, et que l'autre branche du tube (9) est fixée dans une direction longitudinale de l'extrémité du tube (4).

5. Cassette de tube (1) selon une des revendications 1 à 4, **caractérisée en ce que** l'élément circulaire s'étend sur au moins 90°, de préférence sur 170° à 190°, de préférence la plus haute sur 180°.

6. Cassette de tube (1) selon une des revendications 1 à 5, **caractérisée en ce que** le tube (4) est formé au moins dans une région de ce segment circulaire en un matériau élastique en caoutchouc, en particulier en un matériau élastomère naturel ou synthétique, de préférence un caoutchouc silicone ou un polyuréthane-(co)polymère élastomère.

7. Cassette de tube (1) selon une des revendications 1 à 6, **caractérisée en ce que** la branche fixée du tube (9) comprend un élément de jonction (12) ayant des surfaces d'arrêt (13) de préférence s'étendant orthogonalement à une extension longitudinale du tube (4), ces surfaces d'arrêt (13) portant contre des surfaces de fixation (14) dans ce boîtier de la cassette (2).

8. Cassette de tube (1) selon une des revendications 1 à 7, **caractérisée en ce que** l'élément de jonction (12) de la branche fixée du tube (9) comprend un capteur de pression (15) en communication avec la pompe péristaltique (2), si la cassette (1) est montée à la pompe péristaltique (2).

9. Cassette de tube (1) selon une des revendications 2 à 8, **caractérisée en ce que** la branche amovible du tube (10) comprend un élément de jonction (16) avec un élément d'accrochage.

10. Cassette de tube (1) selon la revendication 9, **caractérisée en ce que** cet élément d'accrochage a une surface d'arrêt (17) pour lever un élément de serrage (18), si la cassette (1) est montée à la pompe péristaltique (2), à partir de la position de serrage pendant le déplacement de la branche amovible du tube (10), à partir de la position de montage dans la position de fonctionnement contre une pression de ressort, et que cet élément d'accrochage comprend une surface de blocage (19) voisine à la surface d'arrêt (17) dans la direction de la position de fonctionnement, cet élément de serrage s'engrenant dans sa position de serrage derrière l'élément de serrage (18).

11. Cassette de tube (1) selon la revendication 10, **caractérisée en ce que** l'élément de serrage (18) est configuré comme une goupille de serrage (18).

12. Cassette de tube (1) selon une des revendications 1 à 11, **caractérisée en ce que** le tube (4), le cas échéant communément avec les éléments de jonction (12, 16), est monté de manière enlevable dans le boîtier de la cassette (3).

13. Cassette de tube (1) selon une des revendications 1 à 12, **caractérisée en ce que** le boîtier de la cassette (3) a une forme essentiellement à moitié ovale avec deux faces planes à moitiés ovales, une face à moitié ovale étant fermée et la face à moitié ovale opposée étant ouverte et comprenant l'évidement (5) pour l'accrochage de la roue à galet (6).

14. Cassette de tube (1) selon la revendication 13, **caractérisée en ce qu'**au moins un évidement de liaison de forme (7) est prévu à une paroi (20) orthogonalement à ces faces à moitiés ovales, et qu'à distance de la paroi (20) à l'évidement de liaison de forme (7), au moins un élément de friction (8) est prévu, au moyen de cet élément de friction (8) une jonction de manière libérable à la pompe péristaltique (2) étant établie en déplaçant l'élément de friction (8) dans une direction parallèle à la paroi (20).

15. Pompe péristaltique (2) comprenant un boîtier de pompe (21), une roue à galet (6) faisant saillie d'une paroi extérieure (22) du boîtier de pompe (21) et une cassette (1) selon une des revendications 1 à 13, dans laquelle des éléments de jonction (23, 24) sont prévus à la paroi extérieure (22), ces éléments de jonction étant complémentaires aux éléments de jonction (7, 8) de la cassette.

16. Pompe péristaltique (2) selon la revendication 15, dans laquelle le capteur de pression (15) est configuré comme une chambre de pression avec une membrane de pression (15), et dans laquelle un convertisseur de pression (25) est monté au boîtier de la pompe (21), de sorte qu'avec la cassette (1) étant montée, la membrane de pression (15) porte contre le convertisseur de pression (25).
